# EUROPEAN PATENT APPLICATION

(11) **EP 4 835 467 A1**
(43) Date of publication of application: **04.11.2026**
(21) Application number: 23963128.6
(22) Date of filing: 27.12.2023
(51) Int. Cl.: G01N 33/48, G01N 33/50

(54) **METHOD FOR DETERMINING PLASMA FRACTIONATION CONDITIONS, PLASMA FRACTIONATION DEVICE, AND DNA EXTRACTION DEVICE EQUIPPED WITH SAME**

(71) Applicant: Hitachi High-Tech Corporation, Minato-ku Tokyo 105-6409 (JP)
(72) Inventor: MATSUMOTO, Yuki, Tokyo 105-6409 (JP); FUJIOKA, Michiru, Tokyo 105-6409 (JP); KANDA, Katsuhiro, Tokyo 105-6409 (JP)
(74) Representative: MERH-IP Matias Erny Reichl Hoffmann Patentanwälte PartG mbB
(86) International application number: PCT/JP2023/046974
(87) International publication number: WO 2025/141779

(57) **Abstract**

This method for determining fractionation conditions for plasma 105 includes: a step for measuring a blood sample by means of a blood condition confirmation unit; a step for obtaining a converted number of elapsed days from the blood collection date of the blood sample on the basis of the measurement results; a step for calculating the amount of gDNA in the blood sample on the basis of the converted number of elapsed days; and a step for determining the plasma fractionation conditions on the basis of the calculated amount of gDNA. The present invention thus provides a method for determining plasma fractionation conditions, a plasma fractionation device, and a DNA extraction device equipped with the same, with which it is possible to perform a plasma fractionation step under more appropriate conditions than in the past.

## Description

### Technical Field

The present invention relates to a method for determining plasma fractionation conditions, a plasma fractionation device, and a DNA extraction device equipped with the same, which are capable of setting an appropriate plasma fractionation condition by determining a condition of plasma in a blood collection tube.

### Background Art

As an example of a sample information detection device capable of detecting a condition of a sample with high accuracy and high efficiency, PTL 1 describes a device including an imaging device that images a sample container containing a sample, an illumination device that irradiates the sample container with light from a direction intersecting an imaging direction during imaging, and an information processing unit that detects a condition in the sample container by image processing based on image information of the sample container acquired by imaging the sample container.

### Citation List

### Patent Literature

PTL 1: JP2022-15923A

### Summary of Invention

### Technical Problem

In recent years, it has become clear that peripheral blood of cancer patients contains DNA derived from cancer cells, and this DNA has attracted attention as a target for detecting genetic information of cancer.

A short DNA generated by disruption of cells in the body by apoptosis or the like and fragmentation of a genomic DNA (gDNA) present in the nucleus is called a cell free DNA (cfDNA) in blood. In addition, DNA liberated from cells derived from diseases such as cancer also circulates in blood, and is referred to as cell-tumor DNA (ctDNA) to distinguish it from cfDNA.

A new technique for diagnosing a disease such as cancer by extracting ctDNA from a body fluid such as blood and using the ctDNA as a test target is called a liquid biopsy test.

The liquid biopsy test has an advantage that a test sample can be relatively easily acquired by a minimally invasive method such as blood collection as compared with a biopsy tissue test in which tissue is directly collected during surgery. That is, it is expected that by detecting ctDNA derived from cancer cells in blood, cancer can be detected at an early stage without analyzing cancer cells themselves at a diseased site.

As a workflow of the liquid biopsy test, first, whole blood collected from a patient is centrifuged, and plasma and buffy coat are fractionated. In particular, plasma components are generally obtained by performing centrifugation twice. Next, the ctDNA is extracted from the fractionated plasma, and gDNA is extracted from the buffy coat, and DNA size measurement and quantification are then performed in a quality check (QC) step.

For the DNA obtained in the pretreatment steps from centrifugation to QC, mutation of the gene sequence is analyzed by the next generation sequencing (NGS) or the digital polymerase chain reaction (PCR) (dPCR), and cancer diagnosis and selection of a therapeutic agent are performed.

The genetic test using NGS is a test of detecting mutation in a gene sequence or detecting the amount of mutation by reading a DNA base sequence in a super-parallel manner. As an example of the genetic test using NGS, there is a method of performing cancer diagnosis by separately analyzing a sequence of gDNA which has a normal base sequence and a sequence of ctDNA derived from a cancer cell, and comparing the sequences. Meanwhile, the genetic test using dPCR is a test that detects mutation or measures the amount of mutation by dividing the reaction into several thousands of parallel PCR reactions and amplifying specific gene sequences.

In this workflow, in the step of fractionating plasma, it is required to recover plasma containing a large amount of ctDNA to be subjected to gene mutation analysis without waste while preventing mixture with other components, which is a very important step in advancing the subsequent step.

The condition of the plasma component obtained after centrifugation varies depending on the number of elapsed days from a blood collection date or the type of blood collection tube. In addition, conditions such as fragility and viscosity of buffy coat, which is a white blood cell fraction, also vary similarly to plasma. Therefore, when plasma is fractionated under uniform fractionation conditions, the amount of fractionated plasma is small, and when other components, particularly buffy coat is mixed, gDNA derived from white blood cells is mixed in ctDNA at the stage of nucleic acid extraction. This causes a problem that the mutation detection sensitivity is lowered in NGS, dPCR, and the like.

From the above, it is important to determine the condition of the plasma in the sample in consideration of the number of elapsed days from the blood collection date or the type of the blood collection tube, and to optimize the fractionation conditions according to the plasma condition.

The plasma hemolysis condition is often used as an index for determining the plasma condition. In PTL 1, the hemolysis condition of plasma or serum is determined from the hue using an HSV method. When it is determined that the hemolysis condition exceeds a threshold value, it is determined that the test is not possible because there is sample test inhibition, and the step after fractionating is not performed.

However, the condition of the plasma component after centrifugation varies depending on the number of days after blood collection and the type of blood collection tube. The condition (fragility) of the buffy coat, which is a white blood cell fraction, also varies similarly to the plasma.

Therefore, there is a problem that white blood cells derived from the buffy coat may be mixed when plasma is fractionated under uniform fractionation conditions. That is, since it is considered that gDNA is mixed at the time of extracting ctDNA and the sensitivity decreases in gene mutation analysis such as next generation sequencing (NGS) or digital PCR (dPCR), it is desired to avoid this.

In order to avoid this problem, it is necessary to unify the type of blood collection tube, the number of days after blood collection, and the storage condition to some extent, but it is very difficult to adopt such a measure. Therefore, it is desired to cope with the case of performing the plasma fractionation step on an analysis side, and measures are required.

The invention provides a method for determining plasma fractionation conditions, a plasma fractionation device, and a DNA extraction device equipped with the same, with which it is possible to perform a plasma fractionation step under more appropriate conditions as compared with the related art.

### Solution to Problem

The invention includes a plurality of configurations for solving the above problems, and an example thereof includes: a step of obtaining the number of elapsed days from a blood collection date of a blood sample; a step of calculating an amount of gDNA in the blood sample based on the number of elapsed days; and a step of determining a plasma fractionation condition based on the calculated amount of gDNA.

### Advantageous Effects of Invention

According to the invention, the plasma fractionation step can be performed under more appropriate conditions as compared with the related art. Problems, configurations, and effects other than those described above will be clarified by the following description of embodiments.

### Brief Description of Drawings

[FIG. 1] FIG. 1 is a schematic configuration diagram of a plasma fractionation device according to Embodiment 1 and a DNA extraction device equipped with the same.
[FIG. 2] FIG. 2 is a flowchart illustrating an example of a flow of a method for determining plasma fractionation conditions according to Embodiment 1.
[FIG. 3] FIG. 3 is a diagram illustrating a relation between the number of elapsed days from a blood collection date and absorbance.
[FIG. 4] FIG. 4 is a diagram illustrating a relation between hue and saturation.
[FIG. 5] FIG. 5 is a diagram illustrating a relation between hue and brightness.
[FIG. 6] FIG. 6 is a diagram illustrating a relation between the number of elapsed days from the blood collection date and the amount of gDNA.
[FIG. 7] FIG. 7 is a diagram illustrating details of set fractionation conditions with respect to a threshold value and regions of the amount of gDNA provided in FIG. 6.
[FIG. 8] FIG. 8 is a diagram for examining the presence or absence of gDNA mixed in plasma, the presence or absence of a precipitate after second centrifugation, and the presence or absence of gDNA mixed in the precipitate for two samples having different number of elapsed days from a blood collection date in Embodiment 2.
[FIG. 9] FIG. 9 is a flowchart illustrating an example of a flow of a method for determining plasma fractionation conditions according to Embodiment 3.

### Description of Embodiments

Hereinafter, embodiments of a method for determining plasma fractionation conditions, a plasma fractionation device, and a DNA extraction device equipped with the same according to the invention will be described with reference to the drawings.

In the following embodiments, a configuration (including steps in a flowchart) is not necessarily essential unless otherwise specified or unless clearly considered to be essential in principle.

In the drawings used in the description, the same or corresponding components are denoted by the same or similar reference signs, and repeated description of these components may be omitted.

### <Embodiment 1>

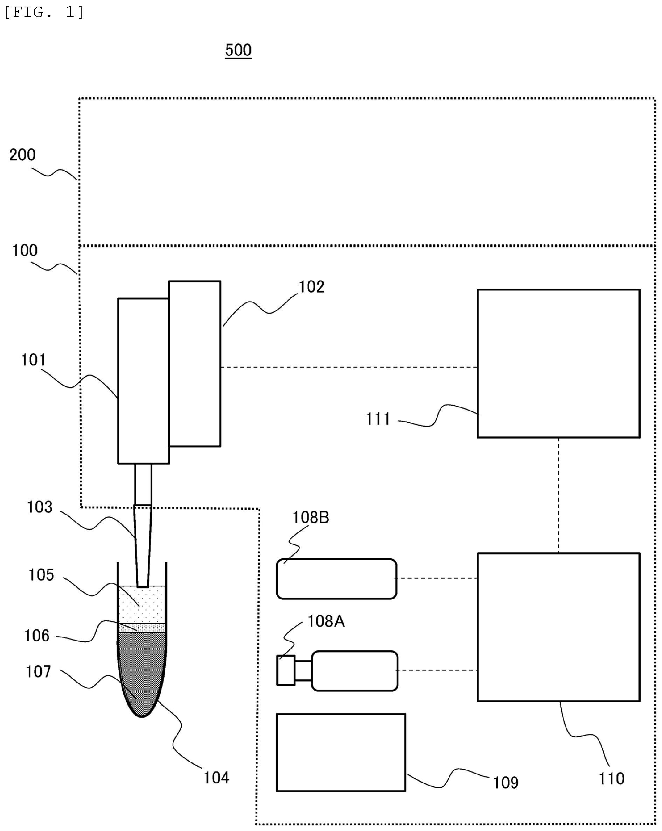

A method for determining plasma fractionation conditions, a plasma fractionation device, and a DNA extraction device equipped with the same according to Embodiment 1 of the invention will be described with reference to FIGS. 1 to 7.

First, overall configurations of a plasma fractionation device and a DNA extraction device, and a fractionation target sample will be described with reference to FIG. 1. FIG. 1 is a schematic configuration diagram of the plasma fractionation device according to Embodiment 1 and the DNA extraction device equipped with the same.

A DNA extraction device 500 illustrated in FIG. 1 includes a plasma fractionation device 100, a nucleic acid extraction unit 200, and the like.

The plasma fractionation device 100 is a device for fractionating plasma 105 from a blood sample, and includes a fractionation machine 101, an actuator 102, a camera 108A, a light receiving unit 108B, an illuminator 109, a motor driver 111, a controller 110, and the like.

The fractionation target sample is a blood sample in a blood collection tube 104 after a whole blood sample obtained by collecting blood from a patient is centrifuged. Blood cell components obtained after centrifuging the blood collection tube 104 containing the blood sample are plasma 105, buffy coat 106, and red blood cells 107 in order from the top layer.

The fractionation machine 101 has a function of fractionating the plasma 105 from the blood sample in the blood collection tube 104, and is designed such that the user fractionates an appropriate amount of the plasma 105 with the controller 110. A fractionation tip 103 is detachably provided at the tip of the fractionation machine 101.

The actuator 102 is a mechanism operated by driving of the motor driver 111, and the height of the fractionation machine 101 can be appropriately adjusted so that the fractionation machine 101 can start aspirating the plasma 105 from an appropriate height and end the aspiration at a height just before the buffy coat 106 layer.

The camera 108A is disposed so as to be able to image the entire blood collection tube 104. When imaging the blood collection tube 104, imaging is performed in a condition in which there is light emitted from the illuminator 109. The light receiving unit 108B is a device that detects transmitted light emitted from the illuminator 109 and transmitted through the blood collection tube 104 or reflected light emitted from the illuminator 109 and reflected from the blood collection tube 104. The camera 108A and the light receiving unit 108B may be integrated.

The illuminator 109 irradiates the blood collection tube 104 with light when the camera 108A images the blood collection tube 104 or when the light receiving unit 108B acquires transmitted light or reflected light of the blood collection tube 104. The light with which the blood collection tube 104 is irradiated is preferably transmitted light or reflected light, but is not limited thereto. In addition, the type of color of light is preferably white light or blue light, but is not limited thereto, similarly to the type of irradiation light.

The camera 108A, the light receiving unit 108B, and the illuminator 109 constitute a blood condition confirmation unit that measures the condition of the blood sample.

The controller 110 is a part that controls operations of the fractionation machine 101, the actuator 102, the camera 108A, the light receiving unit 108B, the illuminator 109, the motor driver 111, and the like, and is implemented as a computer equipped with a display device such as a liquid crystal display, an input device, a storage device including a recording medium such as an HDD or an SSD and its controller, a CPU, a memory, and the like. The control of the operation of each device is executed by the controller 110 based on various programs recorded in the storage device.

Operation control processing to be executed by the controller 110 may be integrated in one program, or may be performed by respective programs or a combination thereof. A part or all of the programs may be implemented by dedicated hardware or may be modularized.

In the present embodiment, the controller 110 obtains a converted number of elapsed days from a blood collection date of the blood sample based on a result of the measurement executed by the camera 108A, the light receiving unit 108B, and the illuminator 109, calculates the amount of gDNA in the blood sample based on the converted number of elapsed days, and determines the plasma fractionation condition based on the calculated amount of gDNA. Details thereof will be described below.

The nucleic acid extraction unit 200 is a mechanism for extracting nucleic acids from the plasma 105 fractionated from the blood sample by the plasma fractionation device 100.

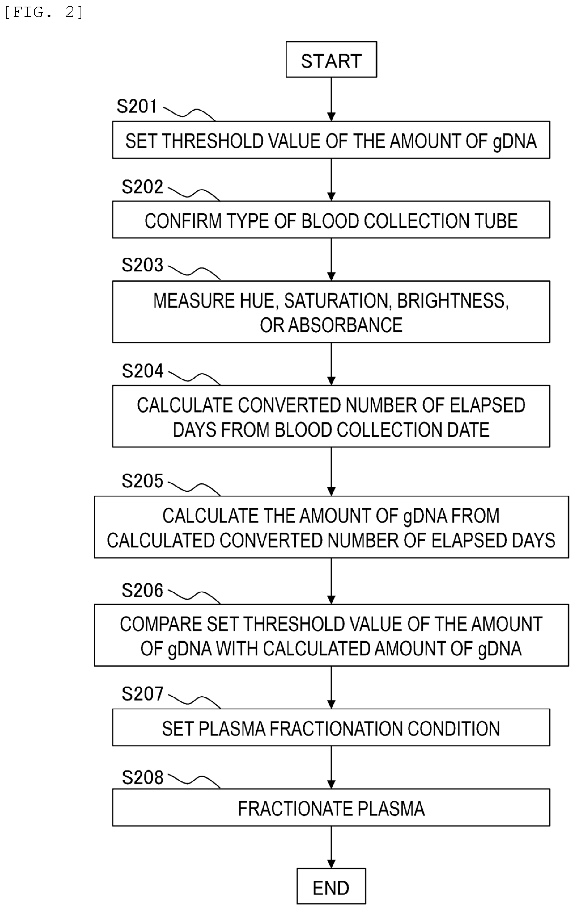

Next, steps until plasma is fractionated from a whole blood sample, which are preferably executed by the plasma fractionation device 100 illustrated in FIG. 1, and a method for determining fractionation conditions for the plasma 105 according to Embodiment 1 of the invention will be described with reference to FIGS. 2 to 7.

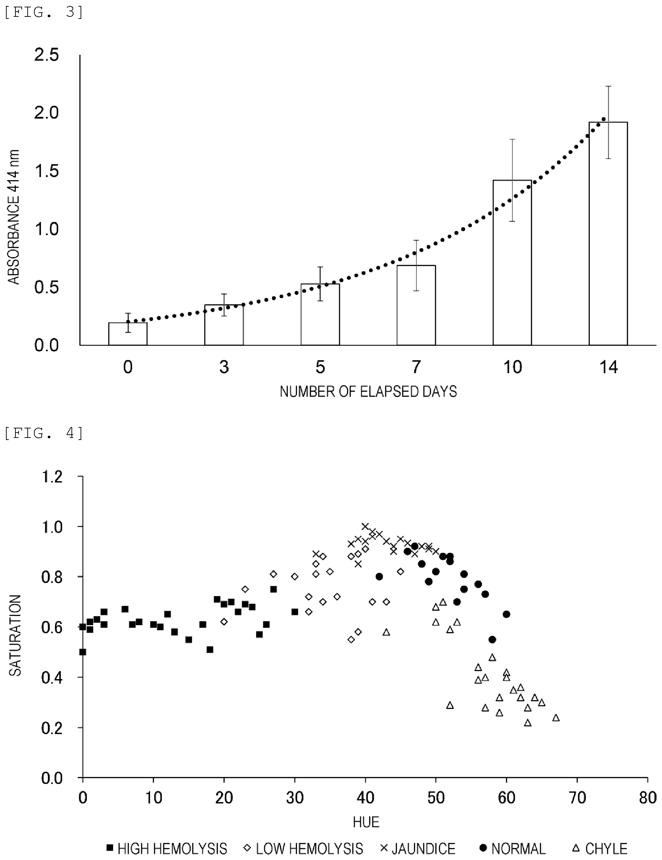

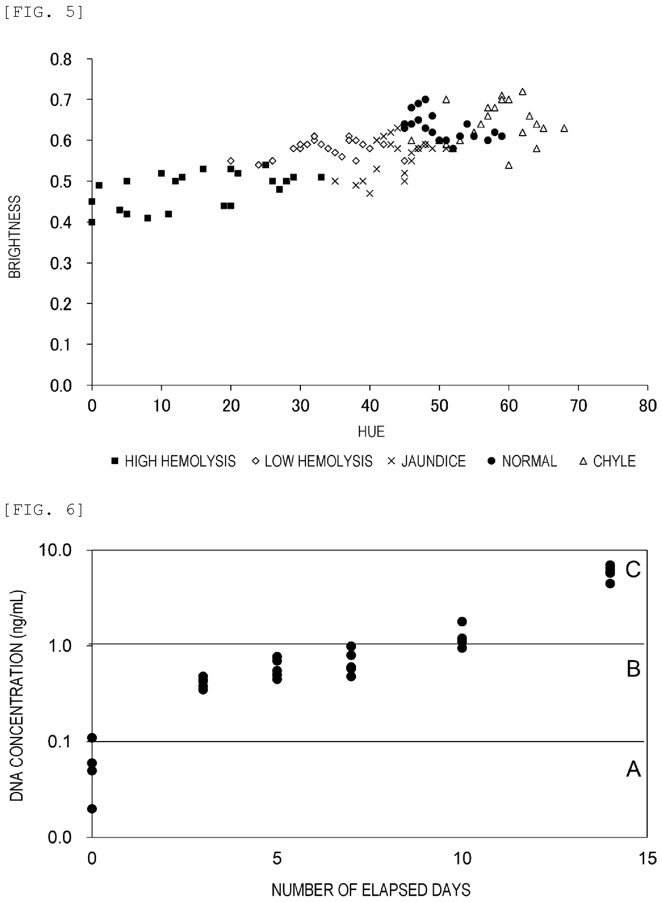

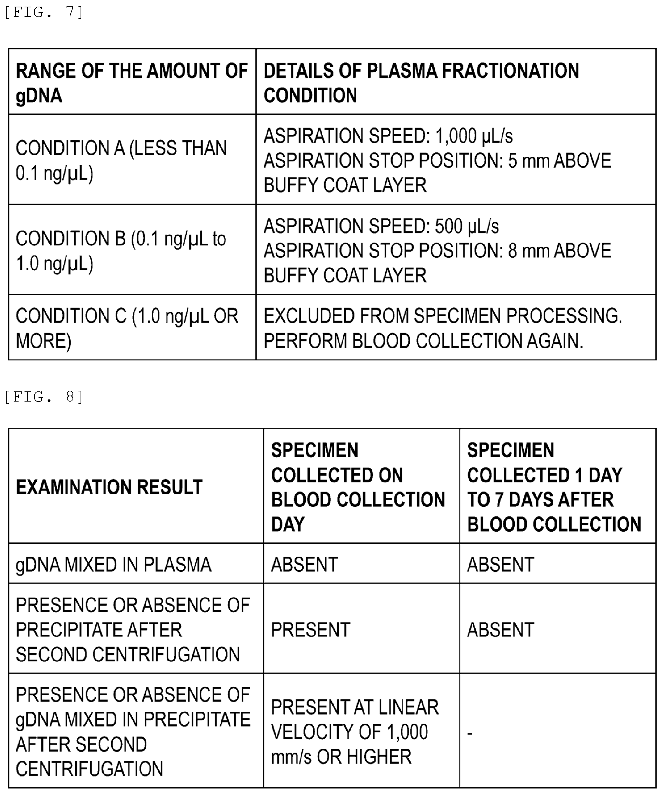

FIG. 2 is a flowchart illustrating an example of a flow of the method for determining plasma fractionation conditions according to Embodiment 1, FIG. 3 is a diagram illustrating a relation between the number of elapsed days from the blood collection date and absorbance, FIG. 4 is a diagram illustrating a relation between hue and saturation, FIG. 5 is a diagram illustrating a relation between hue and brightness, FIG. 6 is a diagram illustrating a relation between the number of elapsed days from the blood collection date and the amount of gDNA, and FIG. 7 is a diagram illustrating details of set fractionation conditions with respect to a threshold value and regions of the amount of gDNA provided in FIG. 6.

As illustrated in FIG. 2, first, the user operates the controller 110 to set a threshold value of the amount of gDNA mixed into the plasma 105 (S201). Step S201 corresponds to a step of setting a threshold value of the amount of gDNA.

Next, the type of the blood collection tube 104 is confirmed (S202). In step S202, the user confirms the type of the blood collection tube 104 and inputs the type to the controller 110. Alternatively, the controller 110 recognizes the type of the blood collection tube 104 using a barcode attached to the blood collection tube 104. Further, other methods may be used.

Since the controller 110 has data indicating the relation between the number of elapsed days from the blood collection date and the amount of gDNA for each type of blood collection tube 104 as illustrated in FIG. 3, and therefore uses data selected in subsequent steps.

Next, the hue, saturation, brightness, or absorbance of the plasma 105 obtained by first centrifugation of the whole blood sample is measured (S203). Step S203 corresponds to a step of measuring the blood sample by the blood condition confirmation unit.

In the method of measuring hue, saturation, and brightness, first, the camera 108A acquires an image of the blood collection tube 104 after the centrifugation step. The type of image acquired at this time is preferably an RGB image. The RGB image is subjected to HSV conversion to detect the boundaries between the plasma 105 layer, the buffy coat 106 layer, and the red blood cell 107 layer, and only the plasma 105 layer is extracted. FIG. 4 illustrates a plot of plasma data diagnosed as high hemolysis, low hemolysis, jaundice, normal, and chyle, according to hue and saturation values, and FIG. 5 illustrates a plot of plasma data diagnosed as high hemolysis, low hemolysis, jaundice, normal, and chyle, according to hue and brightness values.

Here, not only the region of the plasma 105 layer but also the amount of the plasma 105 and the region of the interface between the plasma 105 layer and the buffy coat 106 layer can be determined. Detecting the boundary between the buffy coat 106 layer and the plasma 105 layer is an important step from the viewpoint of preventing gDNA derived from white blood cells in the buffy coat 106 from being mixed into the plasma 105. Based on the extracted plasma 105 layer region, color information including hue, saturation, and brightness of the plasma 105 layer is acquired.

In the absorbance measurement method, incident light from the illuminator 109 is directed onto the plasma 105 layer in the blood collection tube 104, which is a measurement target, and the intensity of the transmitted light is measured by the light receiving unit 108B. The absorbance is calculated from the intensities of each of the incident light and the transmitted light measured by the light receiving unit 108B.

As illustrated in FIG. 3, the absorbance of the plasma 105 layer at a wavelength of 414 [nm] generally increases as the number of elapsed days from the blood collection date increases. It is known that an absorbance peak is observed at 414 [nm] in a condition where hemoglobin contained in red blood cells is bound to oxygen, and a blood condition and a mixed amount of gDNA in the plasma 105 layer can be known using light of this wavelength. Therefore, a higher absorbance indicates a higher blood condition of the plasma 105 and that more days have elapsed from the blood collection date.

At this time, the hue, saturation, and brightness are preferably measured using white light, and the absorbance is preferably measured using blue light having a wavelength of 414 [nm].

As described above, preferably, a blue light source is used as the illuminator 109, the light receiving unit 108B that measures the absorbance of the blood sample is used as the blood condition confirmation unit, and in the measurement step, the absorbance of the blood sample is measured by the illuminator 109 that irradiates the blood sample and the light receiving unit 108B, or preferably, a white light source is used as the illuminator 109, the camera 108A that images the appearance of the blood sample is used as the blood condition confirmation unit, and in the measurement step, any one or more of the hue, saturation, and brightness of the blood sample can be measured by the illuminator 109 that irradiates the blood sample and the camera 108A.

Next, the converted number of elapsed days from the blood collection date is calculated (S204).

For example, it is assumed that data indicating the relation between the number of elapsed days from the blood collection date and the absorbance of the plasma 105 as illustrated in FIG. 3 is recorded in the controller 110, and when the absorbance is measured using blue light in step S203, the converted number of elapsed days from the blood collection date is calculated based on the measured absorbance. When any one or more of the hue, saturation, and brightness is measured using white light in step S203, the converted number of elapsed days from the blood collection date is similarly calculated based on any one or more values of the measured hue, saturation, and brightness.

At this time, the actual number of elapsed days from the blood collection date is not considered. This is because the condition such as the degree of hemolysis of the blood sample in the blood collection tube 104 varies depending on the storage condition among the condition of the plasma 105, and the actual number of days from the blood collection date is low in reliability, so that the converted number of elapsed days is obtained.

Step S204 corresponds to a step of obtaining the converted number of elapsed days from the blood collection date of the blood sample based on the result of the measurement. In the step of obtaining the converted number of elapsed days, the converted number of elapsed days can be calculated using data indicating a relation between the value of the absorbance of the blood sample and the converted number of elapsed days, or the converted number of elapsed days can be calculated using data indicating a relation between any one or more values of the hue, saturation, and brightness and the converted number of elapsed days.

Next, the amount of gDNA is calculated from the calculated converted number of elapsed days (S205). Step S205 corresponds to a step of calculating the amount of gDNA in the blood sample based on the converted number of elapsed days. In addition, in the step of calculating the amount of gDNA, the amount of gDNA can be further obtained by using data indicating a relation between the converted number of elapsed days and a mixed amount of gDNA for each type of the blood collection tube 104 containing the blood sample.

The controller 110 is assumed to have recorded, in addition to the data indicating the relation between the number of elapsed days from the blood collection date and the amount of gDNA as illustrated in FIG. 3 selected in step S202, data indicating the relation between the number of elapsed days from the blood collection date and the amount of gDNA as illustrated in FIG. 6, and using the converted number of elapsed days from the blood collection date calculated in the previous step S204, the controller 110 calculates the amount of gDNA estimated to be actually mixed in the plasma 105.

FIG. 6 illustrates data of the amount of gDNA calculated for each number of elapsed days from the blood collection date by plotting n = 5. Further, each region when the threshold value set in step S201 is set for the amount of gDNA is illustrated. However, this threshold value can be freely set by the user, and is not limited to that illustrated in the drawing.

Next, the set threshold value of the amount of gDNA is compared with the calculated amount of gDNA (S206).

Based on the threshold value of the amount of gDNA set by the user in step S201, the controller 110 compares whether the amount of gDNA calculated in the previous step S205 exceeds the threshold value. When the comparison is completed, the regions for each threshold value illustrated in FIG. 6, that is, the fractionation conditions illustrated in the next step S207 are set. What is illustrated in FIG. 6 is an example, and the invention is not limited thereto.

Next, the plasma fractionation condition is set (S207). Step S207 corresponds to a step of determining plasma fractionation conditions based on the calculated amount of gDNA.

For example, the fractionation condition is set by the controller 110 based on the graph illustrated in FIG. 6. The parameters of the fractionation conditions include a linear velocity (aspiration flow rate) of the plasma 105, a plasma aspiration stop position, a liquid surface tracking descent speed of the fractionation tip 103, and an in-liquid insertion distance of the tip of the fractionation tip 103 as illustrated in FIG. 7.

For example, it is assumed that the user sets the threshold value of the amount of gDNA mixed into the plasma 105 to 1.0 [ng/µL] in step S201.

When the calculated amount of gDNA is actually 1.0 [ng/µL] or more (region C in FIG. 6), the sample is excluded from processing, and the user is instructed to perform blood collection from the patient again.

Next, in a case in which the calculated amount of gDNA is 0.1 [ng/µL] or more and less than 1.0 [ng/µL] (region B in FIG. 6), in order to prevent mixing of gDNA derived from the buffy coat 106, the fractionation conditions are performed under conditions (aspiration speed: 500 [µL/s], aspiration stop position: 8 [mm] above buffy coat 106 layer) gentler than normally used conditions (following condition A).

Further, when the calculated amount of gDNA is less than 0.1 [ng/µL] (region A in FIG. 6), the fractionation is performed under normal fractionation conditions (aspiration speed: 1,000 [µL/s], aspiration stop position: 5 [mm] above buffy coat 106 layer).

As described above, in the step of determining the plasma fractionation condition in step S207, the plasma fractionation condition can be determined by comparing the set threshold value with the calculated amount of gDNA.

The threshold value of the amount of gDNA and the number of provided regions illustrated in this step are merely examples, and the invention is not limited thereto. The details of the fractionation condition can also be set by the user.

Next, the plasma 105 is fractionated according to the fractionation condition (see FIG. 7) set in the previous step S207 (S208). When fractionating the plasma 105, imaging is performed with the camera 108A, or the amount of the plasma 105 is calculated in step S203, so the plasma 105 may be fractionated based on this information.

Next, effects of the present embodiment will be described.

The method for determining fractionation conditions for the plasma 105 according to Embodiment 1 of the invention described above includes a step of obtaining the number of elapsed days from the blood collection date of the blood sample, a step of calculating the amount of gDNA in the blood sample based on the number of elapsed days, and a step of determining the plasma fractionation condition based on the calculated amount of gDNA. In particular, the method further includes a step of measuring the blood sample by the blood condition confirmation unit, and in the step of obtaining the number of elapsed days, the converted number of elapsed days from the blood collection date of the blood sample is obtained as the number of elapsed days based on the result of the measurement, and in the step of calculating the amount of gDNA, the amount of gDNA is calculated based on the converted number of elapsed days.

As described above, by determining the plasma fractionation condition in consideration of the number of days after blood collection and the type of the blood collection tube 104 in terms of the blood condition of the plasma component obtained after centrifugation on the analysis side, it is possible to avoid plasma fractionation in which a large amount of components such as gDNA, which is an inhibition factor for analysis, is mixed and a good test result cannot be obtained, as compared with the related art, without unifying the type of the blood collection tube, the number of days after blood collection, and the storage condition to some extent. Therefore, it is possible to reduce unnecessary nucleic acid extraction and execution of NGS as compared with the related art, and it is possible to avoid waste of costs and test substances and to avoid a time wasted by retesting, that is, it is possible to improve test efficiency as compared with the related art.

In addition, in the step of calculating the amount of gDNA, since the amount of gDNA is further obtained by using data indicating the relation between the converted number of elapsed days and the mixed amount of gDNA for each type of the blood collection tube 104 containing the blood sample, the mixed amount of gDNA can be obtained with higher accuracy, and thus the plasma fractionation conditions can be more appropriately set.

Further, in the step of measuring using the camera 108A configured to image the appearance of the blood sample as the blood condition confirmation unit, any one or more of the hue, the saturation, and the brightness of the blood sample are measured by the illuminator 109 configured to irradiate the blood sample and the camera 108A, and in the step of obtaining the converted number of elapsed days, the converted number of elapsed days is calculated using the data indicating the relation between any one or more values of the hue, the saturation, and the brightness and the converted number of elapsed days, and in particular, by using the white light source as the illuminator 109, it is possible to perform determination using data highly correlated with the mixed amount of gDNA.

In addition, in the step of measuring using the light receiving unit 108B configured to measure the absorbance of the blood sample as the blood condition confirmation unit, the absorbance of the blood sample is measured by the illuminator 109 configured to irradiate the blood sample and the light receiving unit 108B, and in the step of obtaining the converted number of elapsed days, the converted number of elapsed days is calculated using the data indicating the relation between the value of the absorbance of the blood sample and the converted number of elapsed days, and in particular, by using the blue light source as the illuminator 109, it is possible to determine the mixed amount of gDNA using the data highly correlated with the mixed amount of gDNA.

Further, the method further includes a step of setting the threshold value of the amount of gDNA, and in the step of determining the plasma fractionation condition, the set threshold value is compared with the calculated amount of gDNA to determine the plasma fractionation condition, so that it is possible to set the fractionation condition corresponding to the change in the allowable amount of the mixed amount of gDNA according to the subsequent nucleic acid extraction or analysis method, and it is possible to more appropriately execute the sample test.

### <Embodiment 2>

A method for determining plasma fractionation conditions, a plasma fractionation device, and a DNA extraction device equipped with the same according to Embodiment 2 of the invention will be described with reference to FIG. 8.

FIG. 8 is a graph of results of examining the presence or absence of gDNA mixed in plasma, the presence or absence of a precipitate after second centrifugation, and the presence or absence of gDNA mixed in the precipitate for two samples having different number of elapsed days from a blood collection date.

Based on the results illustrated in FIG. 8, it is considered that white blood cells were likely to be mixed in the plasma 105 on the blood collection day, and white blood cells were not mixed in the plasma 105 1 day to 7 days after blood collection. This is caused by the brittleness of the buffy coat 106 layer due to the difference in the number of elapsed days from the blood collection date. That is, fibrin is generated as the number of elapsed days from the blood collection date elapses, but after the first centrifugation, fibrin is mainly accumulated in the buffy coat 106 layer, which is considered to be related to the viscosity and brittleness of the buffy coat 106 layer.

From this result, a condition is assumed that the amount of gDNA calculated in step S206 illustrated in FIG. 2 of Embodiment 1 falls below the threshold value of the amount of gDNA set by the user.

In the present embodiment, in step S207 of FIG. 2, the actual number of days from the blood collection date is also taken into consideration, and with respect to the sample collected on the blood collection date, since the buffy coat 106 layer is brittle and there is a possibility that gDNA derived from white blood cells may be mixed at the time of plasma fractionation, fractionating is performed under a condition more gentler than the normal fractionation condition, for example, the condition B instead of the condition A illustrated in FIG. 7.

Meanwhile, with respect to a sample for which one day or more has elapsed since blood collection date, since the buffy coat 106 layer has not become brittle, plasma fractionation is performed under normal fractionation conditions.

However, the "actual number of days from the blood collection date" used to assign the fractionation condition described in the present embodiment is an example, and the invention is not limited thereto.

Other configurations and operations are substantially the same as those of the method for determining plasma fractionation conditions, the plasma fractionation device, and the DNA extraction device equipped with the same according to Embodiment 1 described above, and details thereof will be omitted.

In the method for determining plasma fractionation conditions, the plasma fractionation device, and the DNA extraction device equipped with the same according to Embodiment 2 of the invention as well, substantially the same effects as those of the method for determining plasma fractionation conditions, the plasma fractionation device, and the DNA extraction device equipped with the same according to Embodiment 1 described above can be obtained.

### <Embodiment 3>

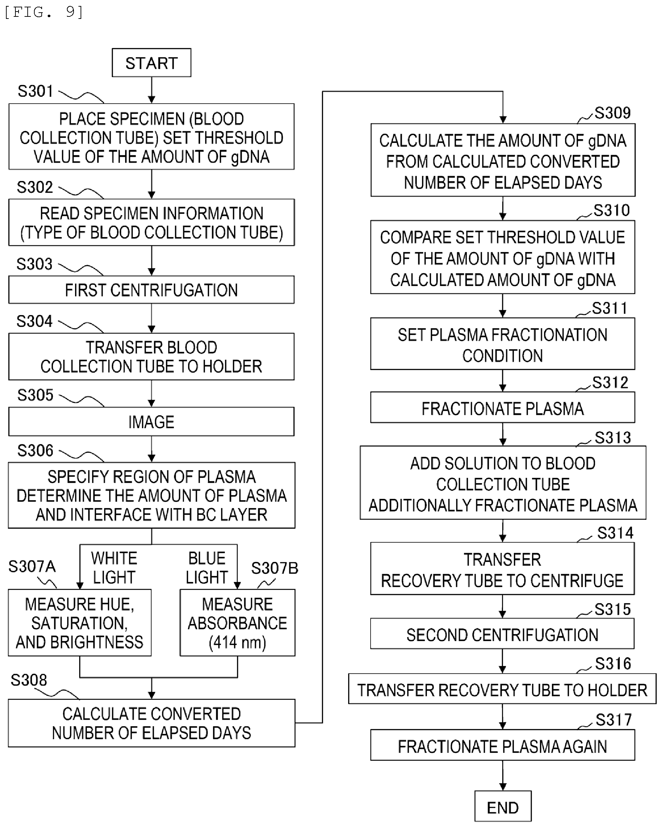

A method for determining plasma fractionation conditions, a plasma fractionation device, and a DNA extraction device equipped with the same according to Embodiment 3 of the invention will be described with reference to FIG. 9. FIG. 9 is a flowchart illustrating an example of a flow of a method for determining plasma fractionation conditions according to Embodiment 3.

In the present embodiment, steps until plasma is fractionated will be described.

First, as illustrated in FIG. 9, a sample (blood collection tube 104) is placed on an information reading unit, and a threshold value of the amount of gDNA is set (S301). Step S301 is substantially the same as step S201 illustrated in FIG. 2.

Next, sample information is read and the information on the type of the blood collection tube 104 is acquired (S302). Step S302 is substantially the same as step S202 illustrated in FIG. 2.

Next, first centrifugation is performed (S303), and the blood collection tube 104 is transferred to a holder (S304), and then the blood collection tube 104 is imaged (S305), and the region of the plasma 105 is specified, the amount of the plasma 105 is determined, and the interface with the buffy coat 106 layer is determined (S306). Although these steps S303 to S306 do not correspond to any steps illustrated in FIG. 2, they are implicitly executed in the flow of FIG. 2 as well.

Next, measurement of hue, saturation, and brightness using white light (S307A) and measurement of absorbance (414 nm) using blue light (S307B) are performed. Step S307A and step S307B are the same as step S203 illustrated in FIG. 2.

Next, a converted number of elapsed days is calculated (S308). Step S308 is substantially the same as step S204 illustrated in FIG. 2.

Next, the amount of gDNA is calculated from the calculated converted number of elapsed days (S309). Step S309 is substantially the same as step S205 illustrated in FIG. 2.

Next, the set threshold value of the amount of gDNA is compared with the calculated amount of gDNA (S310), plasma fractionation conditions are set (S311), and the plasma 105 is fractionated (S312). Step S310 is substantially the same as step S206 illustrated in FIG. 2, step S311 is substantially the same as step S207 illustrated in FIG. 2, and step S312 is substantially the same as step S208 illustrated in FIG. 2.

Next, a solution made of, for example, phosphate buffered saline (PBS) is added to the blood collection tube 104, and the plasma 105 is additionally fractionated (S313).

Specifically, this step is additionally performed on the sample subjected to the plasma fractionation performed in the previous step S312 (see FIG. 7). This step is expected to lead to the recovery of the plasma 105 remaining in the blood collection tube 104 and the recovery of ctDNA in a nucleic acid extraction step.

In step S313, first, the solution is additionally dispensed into the blood collection tube 104 after the plasma fractionation. The solution used at this time is preferably phosphate buffered saline (PBS) (pH 7.4). Since it is known that the general pH of the plasma 105 is around 7 and the density of phosphate buffered saline is about 1 [g/cm³], even if this solution is added, the solution remains in the plasma 105 layer positioned in the uppermost layer and does not affect the physiological activity of the plasma 105 and the degradation of ctDNA. However, the solution is not limited to phosphate buffered saline as long as it remains in the plasma 105 layer and does not affect the physiological activity of the plasma 105 and the degradation of ctDNA.

At the time of solution dispensing, in order to prevent the buffy coat 106 layer and the red blood cell 107 layer, which may be contaminants, from flying up, the solution is added at a reduced dispensing speed, either while the fractionation tip 103 is in contact with a wall surface of the blood collection tube 104 or while the tip of the fractionation tip 103 is in contact with the liquid surface.

Theoretically, a larger amount of the solution to be added is preferable because the plasma 105 can be further recovered. However, since the amount of the solution to be added depends on the amount of the plasma 105 used in the subsequent nucleic acid extraction step, it is necessary to perform adjustment so as to match the amount of the plasma 105 to be additionally fractionated.

After the addition of the solution, plasma fractionation is performed again according to the conditions under which the plasma 105 was fractionated in the previous step S312.

Next, the plasma 105 is fractionated, and the recovery tube is transferred to a centrifugal module (S314) and subjected to centrifugation again (S315). This step is performed to further increase the degree of purification of the plasma 105 fractionated after the first centrifugation.

Next, in order to fractionate the plasma 105 in the recovery tube in which the second centrifugation has been completed in the previous step S315, the recovery tube is transferred to a holder (S316), and in order to prevent a precipitate generated during the second centrifugation from being mixed into the plasma 105, the plasma 105 is fractionated again (S317). In the precipitate generated by the second centrifugation, blood cells such as white blood cells and platelets are observed.

It is desirable that the fractionation conditions for the second fractionation in step S313 and the re-fractionation conditions in step S317 described above are also the same conditions as the fractionation conditions in step S312 (S208) for the first fractionation, that is, the conditions are determined for each sample according to the converted number of elapsed days. In this way, the conditions of the aspiration discharge speed, the aspiration stop position, and the solution addition can be matched.

Other configurations and operations are substantially the same as those of the method for determining plasma fractionation conditions, the plasma fractionation device, and the DNA extraction device equipped with the same according to Embodiment 1 described above, and details thereof will be omitted.

In the method for determining plasma fractionation conditions, the plasma fractionation device, and the DNA extraction device equipped with the same according to Embodiment 3 of the invention as well, substantially the same effects as those of the method for determining plasma fractionation conditions, the plasma fractionation device, and the DNA extraction device equipped with the same according to Embodiment 1 described above can be obtained.

In addition, in the step of determining the plasma fractionation condition, appropriate fractionating and re-fractionating of the plasma 105 according to the condition of the blood sample can be realized by matching the conditions of the aspiration discharge speed, the aspiration stop position, and the solution addition.

### <Others>

The invention is not limited to the embodiments described above, and includes various modifications. The embodiments described above have been described in detail to facilitate understanding of the invention, and the invention is not necessarily limited to those including all the configurations described above.

A part of a configuration in one embodiment can be replaced with a configuration in another embodiment, and a configuration in one embodiment can also be added to a configuration in another embodiment. Another configuration may be added to a part of a configuration in each embodiment, and a part of a configuration in each embodiment may also be deleted or replaced with another configuration.

### Reference Signs List

100: plasma fractionation device
101: fractionation machine
102: actuator
103: fractionation tip
104: blood collection tube
105: plasma
106: buffy coat
107: red blood cell
108A: camera (blood condition confirmation unit, imaging device)
108B: light receiving unit (blood condition confirmation unit)
109: illuminator (blood condition confirmation unit, light source)
110: controller
111: motor driver
200: nucleic acid extraction unit
500: DNA extraction device

## Claims

1. A method for determining plasma fractionation conditions comprising:
a step of obtaining the number of elapsed days from a blood collection date of a blood sample;
a step of calculating an amount of gDNA in the blood sample based on the number of elapsed days; and
a step of determining a plasma fractionation condition based on the calculated amount of gDNA.

2. The method for determining plasma fractionation conditions according to claim 1, further comprising:
a step of measuring the blood sample by a blood condition confirmation unit, wherein
in the step of obtaining the number of elapsed days, a converted number of elapsed days from the blood collection date of the blood sample is obtained as the number of elapsed days based on a result of the measurement, and
in the step of calculating the amount of gDNA, the amount of gDNA is calculated based on the converted number of elapsed days.

3. The method for determining plasma fractionation conditions according to claim 2, wherein
in the step of calculating the amount of gDNA, the amount of gDNA is further obtained by using data indicating a relation between the converted number of elapsed days and a mixed amount of gDNA for each type of blood collection tube containing the blood sample.

4. The method for determining plasma fractionation conditions according to claim 2, wherein
an imaging device configured to image an appearance of the blood sample is used as the blood condition confirmation unit,
in the measurement step, any one or more of hue, saturation, and brightness of the blood sample is measured using a light source configured to irradiate the blood sample and the imaging device, and
in the step of obtaining the converted number of elapsed days, the converted number of elapsed days is calculated using data indicating a relation between any one or more values of the hue, saturation, and brightness and the converted number of elapsed days.

5. The method for determining plasma fractionation conditions according to claim 4, wherein
a white light source is used as the light source.

6. The method for determining plasma fractionation conditions according to claim 2, wherein
an absorbance measurement device configured to measure absorbance of the blood sample is used as the blood condition confirmation unit,
in the measurement step, the absorbance of the blood sample is measured by a light source configured to irradiate the blood sample and the absorbance measurement device, and
in the step of obtaining the converted number of elapsed days, the converted number of elapsed days is calculated using data indicating a relation between a value of the absorbance of the blood sample and the converted number of elapsed days.

7. The method for determining plasma fractionation conditions according to claim 6, wherein
a blue light source is used as the light source.

8. The method for determining plasma fractionation conditions according to claim 2, wherein
in the step of determining the plasma fractionation condition, conditions of an aspiration discharge speed, an aspiration stop position, and a solution addition are matched.

9. The method for determining plasma fractionation conditions according to claim 2, further comprising:
a step of setting a threshold value of the amount of gDNA, wherein
in the step of determining the plasma fractionation condition, the set threshold value is compared with the calculated amount of gDNA to determine the plasma fractionation condition.

10. A plasma fractionation device for fractionating plasma from a blood sample, the plasma fractionation device comprising:
a fractionation machine configured to fractionate the plasma from the blood sample; and
a controller configured to obtain the number of elapsed days from a blood collection date of the blood sample, calculate an amount of gDNA in the blood sample based on the number of elapsed days, and determine a plasma fractionation condition based on the calculated amount of gDNA.

11. The plasma fractionation device according to claim 10, further comprising:
a blood condition confirmation unit configured to measure a condition of the blood sample, wherein
the controller is configured to obtain a converted number of elapsed days from the blood collection date of the blood sample as the number of elapsed days based on a result of the measurement executed by the blood condition confirmation unit, and calculate the amount of gDNA in the blood sample based on the converted number of elapsed days.

12. A DNA extraction device comprising the plasma fractionation device according to claim 10.
